Europäisches Patentamt

(19) European Patent Office

Office européen des brevets

(11) Publication number: 0 321 094
A2

## (12) EUROPEAN PATENT APPLICATION

(21) Application number: 88310801.1

(22) Date of filing: 16.11.88

(51) Int. Cl.4: C07K 7/10 , A61K 37/02 , G01N 33/68 , A61M 1/36

(30) Priority: 16.11.87 US 121217

(43) Date of publication of application:
21.06.89 Bulletin 89/25

(84) Designated Contracting States:
AT BE CH DE ES FR GB GR IT LI LU NL SE

(71) Applicant: SCRIPPS CLINIC AND RESEARCH FOUNDATION
10666 North Torrey Pines Road
La Jolla California 92037-1093(US)

(72) Inventor: Zimmerman, Theodore S.
544 Bonair Street
La Jolla California 92037(US)
Inventor: Fulcher, Carol A.
7418 Fay Avenue
La Jolla California 92037(US)

(74) Representative: Jump, Timothy John Simon et al
VENNER, SHIPLEY & CO. 368 City Road
London EC1V 2QA(GB)

(54) Treatment of factor VIII inhibitors.

(57) Fragments of human Factor VIII:C which bind to antibody inhibitors of Factor VIII in patients afflicted with such inhibitors are disclosed. The method of treating Factor VIII inhibitors, by administering one or more of these fragments, is also disclosed.

EP 0 321 094 A2

## TREATMENT OF FACTOR VIII INHIBITORS

This invention relates to the treatment of patients who exhibit Factor VIII inhibitors.

The conventional treatment for hemophilia-A is administration of Factor VIII (antihemophilic factor, or "AHF"), concentrated in any of various means from the plasma of donors. Some hemophiliacs exhibit, in effect, "resistance" to this treatment, in that administration of Factor VIII in doses which are usually effective for most hemophiliacs produces reduced or no therapeutic effect. In addition, persons who are not hemophiliacs can develop these inhibitors.

This phenomenon has generally been considered to be due to one or more anti-Factor VIII antibody inhibitors of Factor VIII in the circulatory system of the afflicted individual. It may be possible to treat Factor VIII inhibitors by administering greatly increased amounts of Factor VIII, some of which serves to saturate the inhibitory capacity of the Factor VIII inhibitor and the remainder of which provides the desired therapeutic effect uninhibited. However, this approach is extremely expensive, consumes large amounts of AHF per patient, and carries the risk of increasing the amounts of other products or agents, unavoidably present with the Factor VIII, to which the patient is exposed.

Thus, there is a need for a product which effectively neutralizes the activity of Factor VIII inhibitors, without introducing unnecessary co-products.

The present invention relates to a polypeptide having the amino acid sequence of the fragment of human Factor VIII containing amino acid residues 1-2332 or a smaller fragment thereof. Thus the polypeptide may have the amino acid sequence of the fragment of human Factor VIII containing amino acid residues 1690-2332 or a portion thereof. The polypeptide may also comprise the amino acid sequence of the fragment of human Factor VIII containing amino acid residues 373-740 or a portion thereof. The polypeptide may have the amino acid sequence of the fragment of human Factor VIII containing amino acid residues 1649-2332 or a portion thereof. In addition, the polypeptide may have the amino acid sequence of the fragment of human Factor VIII containing amino acid residues 1-740 or a portion thereof. The present invention may finally comprise fragments of human Factor VIII containing any of these polypeptides. These polypeptides have been found to react immunologically with Factor VIII inhibitors.

The invention further relates to a polypeptide which neutralizes the activity of Factor VIII inhibitor and whose amino acid sequence comprises that of a fragment of human Factor VIII selected from the group consisting of:

(i) fragments whose amino-terminal residue is one of residues 320-340 and whose carboxyl-terminal residues is one of residues 365-380;

(ii) fragments whose amino-terminal residue is one of residues 360-380 and whose carboxyl-terminal residue is one of residues 394-465;

(iii) fragments whose amino-terminal residue is one of residues 395-466 and whose carboxyl-terminal residue is one of residues 472-492;

(iv) fragments whose amino-terminal residue is one of residues 360-380 and whose carboxyl-terminal residue is one of residues 472-492;

(v) fragments whose amino-terminal residue is one of residues 1674-1694 and whose carboxyl-terminal residue is one of residues 1708-1775;

(vi) fragments whose amino-terminal residue is one of residues 1709-1776 and whose carboxyl-terminal residue is one of residues 1782-1802; and

(vii) fragments whose amino-terminal residue is one of residues 1674-1694 and whose carboxyl-terminal residue is one of residues 1782-1802.

Particularly preferred are polypeptides whose amino acid sequences are those of fragments of human Factor VIII having any of the following sequences:

(i) amino acid residues 340-354;
(ii) amino acid residues 342-356;
(iii) amino acid residues 351-365;
(iv) amino acid residues 380-394;
(v) amino acid residues 466-472;
(vi) amino acid residues 380-472;
(vii) amino acid residues 1694-1708;
(viii) amino acid residues 1776-1782; or
(ix) amino acid residues 1694-1782;

The invention further comprises the method of suppressing or neutralizing Factor VIII inhibitors in a patient who exhibits such inhibitors, by administering to the patient an effective amount of one or more of these polypeptides.

When amino acid residues of human Factor VIII are referred to herein by number, the numbering of the amino acid residues is that reported by Vehar, G. A., et al., "Structure of human factor VIII" in Nature, Vol. 312: 337-342 (1984).

For instance, the polypeptide whose amino acid sequence is that of the fragment having residues 340-354 is:
$H_2N$-Asn-Glu-Glu-Ala-Glu-Asp-Glu-Asp-Tyr-Asp-Asp-Asp-Leu-Thr-Asp-Ser-Glu-COOH.
The polypeptide whose sequence is residues 342-356 is:

H₂N-Glu-Ala-Glu-Asp-Tyr-Asp-Asp-Asp-Leu-Thr-Asp-Ser-Glu-Met-Asp-COOH.

The polypeptide whose sequence is residues 351-363 is: H₂N-Thr-Asp-Ser-Glu-Met-Asp-Val-Val-Arg-Phe-Asp-Asp-Asp-Asn-Ser-COOH.

The polypeptide whose amino acid sequence is that of the fragment having residues 380-394 is: H₂N-Lys-Thr-Trp-Val-His-Tyr-Ile-Ala-Ala-Glu-Glu-Glu-Asp-Trp-Asp-COOH.

The polypeptide whose sequence is residues 466-472 is: H₂N-Lys-Asn-Gln-Ala-Ser-Arg-Pro-COOH.

The polypeptide whose sequence is residues 1694-1708 is:
H₂N-Lys-Thr-Arg-His-Tyr-Phe-Ile-Ala-Ala-Val-Glu-Arg-Leu-Trp-Asp-COOH.

The polypeptide whose sequence is residues 1776-1782 is:
H₂N-Arg-Asn-Gln-Ala-Ser-Arg-Pro-COOH.

The present invention encompasses these polypeptides of interest, as well as polypeptides which contain the amino acid sequences of these polypeptides of interest plus flanking residues at one or both ends thereof. When we refer to the "flanking residues" at an end of a given fragment, we mean the residue(s) immediately adjacent to that end of the fragment, in the sequence in which they appear in the published sequence reported for the Factor VIII polypeptide. For instance, the single flanking residues of the polypeptide whose sequence is that of the fragment of Factor VIII containing residues 466-472 are residues 465 at the amino-terminal end, and 473 at the carboxyl-terminal end. The five flanking residues are residues 461-465 at the amino-terminal end, and residues 473-477 at the carboxyl-terminal end. In addition to the polypeptides of interest described above, polypeptides incorporating any of those sequences and, at either end or at each end, up to 20 flanking residues, or up to 10, or even 1-5 flanking residues, are also of interest in this invention.

The fragment of Factor VIII containing amino acid residues 1690-2332 is the fragment of M about 72,000 described in our earlier U.S. patent applications Serial No. 481,105, Serial No. 556,508, Serial No. 673,916, and Serial No. 738,134, and, European Patent application No. 0202853 the contents of which are hereby incorporated by reference. This fragment is formed by thrombin-induced proteolysis of the fragment containing amino acid residues 1649-2332 of Mr about 80,000 described therein. The fragment containing amino acid residues 373-740 is the fragment of Mr about 44,000 described in those earlier applications. This fragment is formed by thrombin-induced proteolysis of the fragment containing amino acid residues 1-740 of Mr about 92,000 described therein.

The fragments of the present invention can be formed by solid-state synthesis or by recombinant DNA techniques.

In the well-known procedure for solid state synthesis of a polypeptide, the desired polypeptide is assembled starting from an insoluble support such as benzhydryl amine or chloromethylated resin (derived from cross-linked polystyrene, and available from chemical supply houses). The amino acid at the carboxyl-terminal end of the desired polypeptide, carrying protecting groups on the alpha-amino nitrogen and on any other reactive sites, is attached to the resin from solution using known peptide coupling techniques. The protecting group on the alpha-amino group is removed (leaving other protecting groups, if any, intact), and the next amino acid of the desired sequence (carrying suitable protecting groups) is attached, and so on. When the desired polypeptide has been completely built up, it is cleaved from the resin support, all protecting groups are removed, and the polypeptide is recovered. Examples of suitable protecting groups are: alpha-tert-butyloxycarbonyl for the alpha-amino-group; benzyl, 4-methoxybenzyl, or 4-methylbenzyl for the thiol group of cysteine, the beta-carboxylic acid group of aspartic acid, the gamma-carboxylic acid group of glutamic acid, and the hydroxyl groups of serine, threonine, and tyrosine; benzyloxycarbonyl or a 2-chloro- or 3,4-dimethoxy-derivative thereof for the ring nitrogens of histidine and tryptophan and the epsilon-amino group of lysine; p-nitrophenyl for the amide nitrogens of asparagine and glutamine; and nitro or tosyl for the guanidine group of arginine.

To make the desired polypeptide by recombinant DNA techniques, the portion of the gene for human Factor VIII that codes for the fragment of interest is cloned, inserted into a cell, and used to express the fragment. For a description of the gene and how to use it, see Gitschier, J., et al., "Characterization of the human factor VIII gene", Nature, Vol. 312, pp. 326-330; Wood, William I., et al., "Expression of active human factor VIII from recombinant DNA clones", Nature, Vol. 312, pp. 330-337; and Toole, John J., et al., "Molecular cloning of a cDNA encoding human antihemophilic factor", Nature, Vol. 312, pp. 342-347.

The manner of making the fragments having Mr values of about 92,000, about 80,000, about 72,000 and about 44,000, as described in our earlier applications is summarized as follows. Factor VIII is purified and concentrated, preferably to a high degree such as by the process described in our U.S. Patent No. 4,361,509. In that process, Factor VIII (termed Factor VIII:C in that patent) and Factor VIII:RP are adsorbed together from a source such as commercial AHF concentrate onto a monoclonal antibody to Factor VIII:RP which is bound to a solid substrate such as cross-linked agarose (e.g. Sepharose). The other source materials are eluted, and then the Factor VIII is eluted and passed through a second column such as aminohexyl agarose to further purify and concentrate the Factor VIII.

The resulting highly purified Factor VIII is then digested with alpha-thrombin under conditions effective to react with the Factor VIII to form the desired fragments, without completely digesting the Factor VIII. For instance, 200 to 400 units/ml of Factor VIII and 0.1 to 0.5 units/ml of alpha-thrombin can be combined at room temperature in an aqueous system, buffered to a pH of about 6.8 to 7.4. After the reaction has been allowed to proceed long enough for the desired fragment (or fragments) to have formed, the digestion is discontinued by addition of a product which irreversibly inhibits the alpha-thrombin without degrading the Factor VIII fragments. A highly satisfactory product is (p-amidino-phenyl) methanesulfonyl fluoride ("p-APMSF"), in an amount on the order of 1.0 micromoles to 2.5 millimoles per unit of alpha-thrombin initially present. The appropriate period of reaction time before addition of the thrombin inhibitor depends somewhat on which fragment is desired. In general, the fragments having $M_r$ values of about 92,000, about 80.000 and about 72,000 are present as little as 0.1 minute after addition of the alpha-thrombin to the Factor VIII, and remain present for as long as 60 minutes or more of reaction time. The fragment of $M_r$ about 44,000 may form as quickly, but is likely to be present in a greater proportion after about 2 minutes of digestion, through about 60 minutes or longer.

Following inactivation of the alpha-thrombin, the resulting mixture of Factor VIII fragments is treated to recover the desired fragment(s) by conventional techniques for concentration and recovery of polypeptides. Useful techniques include ultrafiltration, ultracentrifugation, ion exchange, gel permeation chromatography, preparative electrophoresis, isoelectric focusing, and gel and affinity chromatography (including affinity chromatography using an antibody to the desired fragment). In particular, sodium dodecyl sulfate-polyacrylamide gel electrophoresis, preferably the Procedure A described in our application Serial No. 481,105 and incorporated herein by reference, can be used to separate the mixture of fragments into discrete bands (each of which contains a different fragment). One or more of the above techniques are used to recover each fragment, preferably free of other non-Factor VIII products.

Each fragment can be recovered as an aqueous solution which may be treated to remove water therefrom by procedures well known in the art. For instance, the mixture can be freeze-dried, or ultrafiltered and then freeze-dried.

The dried compositions containing one or more of the polypeptides of the present invention can be formulated into pharmaceutical preparations for therapeutic, diagnostic, or other uses. To prepare them for intravenous administration, the compositions are dissolved in water containing physiologically compatible substances such as sodium chloride (e.g. 0.35-2.0 M), glycine, and the like and having a buffered pH compatible with physiological conditions. The amount to administer will depend on the severity with which the patient is afflicted with Factor VIII inhibitors, but can be determined readily for any particular patient. Sufficient neutralizer of the inhibitor should be given to reduce or eliminate the inhibition of the effect of a subsequently administered preparation containing Factor VIII.

The Factor VIII fragments of amino acid residues 1649-2332, of amino acid residues 1-740, of amino acid residues 1690-2332, and of amino acid residues 373-740, have been found to bind inhibitors of Factor VIII inhibitor in plasma obtained from patients known to exhibit Factor VIII inhibitory activity. This indicates that a Factor VIII fragment which contains either of those fragments would also neutralize Factor VIII inhibitor activity, and could be administered to suppress a patient's production of Factor VIII inhibitors. The present invention also encompasses fragments within those longer fragments which neutralize Factor VIII inhibitor activity.

Factor VIII inhibitor neutralizing polypeptides having the amino acid sequence of any of these fragments are also useful in diagnosing the presence of Factor VIII inhibitors.

In general, the polypeptide is contacted with a sample of the plasma or blood being tested, and the mixture is then analyzed to determined whether the polypeptide has reacted with an antibody inhibitor. Such a process can employ, for instance, the technique known as "Western blotting". For example, a Factor VIII inhibitor neutralizing polypeptide is transferred by known techniques to a nitrocellulose sheet, where it is immobilized. By "immobilized" we mean fixed or bonded to a solid support so that the protein can be contacted with successive liquid reagent samples without being washed out of the substrate. A sheet such as nitrocellulose is of course preferred as it facilitates the autoradiography step. Immobilization can also be by chemically coupling to agarose beads or other supporting substrates.

The immobilized polypeptide is then washed with the sample being tested. To test whether any protein in the sample has reacted (immunologically) with the immobilized polypeptide, (and thereby become bound to it) the latter is contacted (e.g. washed) and monoclonal antibodies to human IgG (such as 0.25 ug/ml of one or more of mouse monoclonal antibodies to human IgG-1, IgG-2, IgG-3 or IgG-4 as for instance 0.01% processed ascites fluid (Miles Scientific, Naperville, Illinois)). Then, the immobilized polypeptide is visualized by reacting it with e.g. $^{125}$I-labeled rabbit anti-mouse IgG (or another labeled purified antibody to the anti-human IgG used in the previous step) and then subjecting it to autoradiography in known fashion. The presence of a band indicates the presence of Factor VIII inhibitor in the tested plasma.

The following examples are given as illustrative of the present invention. The present invention is not restricted only to these examples.

Example 1

Removal of Factor VIII Antibody Inhibitors From Plasma of a Patient

Plasma from the patient is obtained by using an ordinary cell centrifuge. Blood is drawn at a rate of about 20 ml/min, and is immediately citrated (sodium citrate 0.13M) at a ratio of 1:10 to 1:19. The patient himself is not heparinized. To obtain an even balance on outgoing and ingoing fluid volume, a pumping monitor (BMM 10/ITM, Gambro AB, Lund, Sweden) with safety devices such as ultrasonic air detectors and pressure gauges is used. The cell separator is connected on line to a plasma treatment monitor (CITEM 5), Gambro AB), which controls the separation and removal of Factor VIII antibody inhibitors. The system includes two columns each containing 62.5 ml Sepharose 6MB with a purified, immobilized Factor VIII inhibitor neutralizing polypeptide having the amino acid residues 1-740. The monitor passes the plasma to be treated through one column and simultaneously elutes the other with a citrate-phosphate-gradient (pH from 7.2 to 2.4). The column is then neutralized with sodium chloride acetate solution, pH 7.4. Analysis devices for pH and UV are placed in each line to detect pH changes and plasma protein concentrations. The treated plasma is re-transfused with the patient's red cells.

Example 2

Removal of Factor VIII Antibody Inhibitors From Plasma of a Patient

Blood is drawn from a cubital vein at a rate of 10 to 20 ml/min. It is immediately citrated. An immunotherapy monitor (BMM 10/ITM, Gambro AB) is used for control of the extracorporeal blood flow and the addition of sodium citrate (0.13M) at a ratio fo 1:15. The whole blood is depleted from antibodies by being passed over two columns, each containing 62.5 ml sepharaose 6MB with a purified, immobilized Factor VIII inhibitor neutralizing polypeptide having the amino acid residues 1-740. The blood is then re-transfused.

Example 3

Neutralization of Inhibitor Antibodies In Plasma of a Patient

Three of the peptides deriving from Factor VIII sequences were tested in vitro and shown to partially neutralize the ability of two different human Factor VIII inhibitor antibodies to inactivate Factor VIII. The peptides representing amino acid residues 340-354 and 342-356 were shown to partially block the ability of one of these inhibitor antibodies to inactivate Factor VIII, whereas amino acid residue 351-365 partially blocked a second inhibitor.

In a typical experiment normal human plasma (50ul) was diluted with 50ul of barbital buffered saline and incubated overnight at room temperature as a control sample. Factor VIII activity in the plasma was then measured in an activated partial thromboplastin time assay using an MLA electra 700 automatic coagulation timer. In this assay, the shorter the clotting time, the more Factor VIII is present in the test mixture. Testing of the control sample resulted in a clotting time of 65.4 and 63.9 seconds in duplicate assays. This represents the amount of Factor VIII activity remaining if no Factor VIII was inactivated by inhibitor antibody. When the same test was run with diluted inhibitor plasma and barbital saline it resulted in clotting times of 91.6 and 90.4 seconds. This prolongation of the clotting time represents inactivation of Factor VIII by the inhibitor antibody.

However, if the diluted inhibitor plasma was first incubated with an equal volume of 5 or 10 mM of residue 351-365 in barbital saline overnight at room temperature, then incubated for another 2 hours, with an equal volume of normal plasma, the Factor VIII assay clotting time was reduced to 70.3 or 70.9 seconds. This indicated that amino acid residue 351-365 had partially blocked the ability of the inhibitor antibody to inactivate Factor VIII.

A second inhibitor antibody, not blocked by amino acid residue 351-365 was shown to be partially blocked by both residue 340-354 and 342-365. The clotting time of the plasma containing the second inhibitor antibody was 87.3 and 84.0 seconds. However, when the diluted inhibitor plasma was first incubated with an equal volume of 10mM of residue 340-354 or 343-356, the clotting time was reduced to 76.1 and 73.1 seconds, respectively. This indicated that residues 340-354 and 342-356 had partially blocked the ability of the inhibitor antibody to inactivate Factor VIII.

This shows that these peptides, as well as the others diclosed in the present invention, could be used, alone or possibly in combination with one or more additonal Factor VIII inhibitor antibody neutralizing peptides or fragments to treat Factor VIII inhibitors.

## Claims

1. A polypeptide which neutralizes the activity of Factor VIII inhibitor having an amino acid sequence comprising that of a fragment of human Factor VIII containing amino acid residues 1-2332 or a portion thereof.

2. A polypeptide as claimed in Claim 1 whose amino acid sequence comprises that of a fragment of human Factor VIII selected from the group consisting of:

(i) fragments whose amino-terminal residue is one of residues 320-340 and whose carboxyl-terminal residue is one of residues 365-380;

(ii) fragments whose amino-terminal residue is one of residues 360-380 and whose carboxyl-terminal residue is one of residues 394-465;

(iv) fragments whose amino-terminal residue is one of residues 395-466 and whose carboxyl-terminal residue is one of residues 472-492;

(v) fragments whose amino-terminal residue is one of residues 360-380 and whose carboxyl-terminal residue is one of residues 472-492;

(vi) fragments whose amino-terminal residue is one of residues 1674-1694 and whose carboxyl-terminal residue is one of residues 1708-1775;

(vii) fragments whose amino-terminal residue is one of residues 1709-1776 and whose carboxyl-terminal residue is one of residues 1782-1802; and

(viii) fragments whose amino-terminal residue is one of residues 1674-1694 and whose carboxyl-terminal residue is one of residues 1782-1802.

3. A polypeptide as claimed in Claim 1 whose amino acid sequence comprises that of a fragment of human Factor VIII selected from the group consisting of

(i) amino acid residues 340-354;

(ii) amino acid residues 342-356;

(iii) amino acid residues 351-365;

(iv) amino acid residues 380-394;

(v) amino acid residues 466-472;

(vi) amino acid residues 380-472;

(vii) amino acid residues 1694-1708;

(viii) amino acid residues 1776-1782; or

(ix) amino acid residues 1694-1782;

4. Factor VIII inhibitor neutralizing polypeptide whose amino acid sequence comprises that of the fragment of human Factor VIII composed of amino acid residues 1690-2332 or a smaller fragment thereof.

5. A Factor VIII inhibitor neutralizing polypeptide as claimed in Claim 1 whose amino acid sequence comprises that of the gragment of human Factor VIII composed of amino acid residues 373-740 or a smaller fragment thereof.

6. A Factor VIII inhibitor neutralizing polypeptide as claimed in Claim 1 whose amino acid sequence comprises that of the fragment of human Factor VIII composed of amino acid residues 1649-2332 or a smaller fragment thereof.

7. A Factor VIII inhibitor neutralizing polypeptide as claimed in Claim 1 whose amino acid sequence comprises is that of the fragment of human Factor VIII composed of amino acid residues 1-740 or a smaller fragment thereof.

8. A Factor VIII inhibitor neutralizing product comprising polypeptides as claimed in Claim 1 whose amino acid sequences are those of the fragments of human Factor VIII composed of amino acid residues 1649-2332 or a smaller fragment thereof, amino acid residues 1-740 or a smaller fragment thereof amino acid residues 1690-2332 or a samller fragment thereof and amino acid residues 373-740 or a smaller fragment thereof.

9. A therapeutic composition comprising an amount of one or more polypeptides or products as claimed in any of Claims 1-8 effective to neutralize Factor VIII inhibitor activity in a patient exhibiting such activity, in association with a pharmaceutically acceptable carrier.

10. A method of alleviating Factor VIII inhibitory activity in a patient suffering therefrom, comprising administering to such patient an effective amount of one or more polypeptides according to any of claims 1-8, one or more product according to claim 8, one or more composition as claimed in claim 9, or a mixture thereof.

11. A method for detecting the presence of an antibody inhibitor of human Factor VIII in a sample, comprising

(a) immobilizing on a solid substrate a polypeptide according to any of claims 1-7;

(b) contacting the immobilized polypeptide with the sample; and then

(c) analyzing the immobilized polypeptide to determine whether it has bound an antibody inhibitor.

12. The method of claim 11 wherein step (c) comprises

(1) reacting the immobilized polypeptide with a monoclonal antibody to human IgG; then

(2) reacting the antibody used in step (1) with a radiolabeled antibody thereto; and

(3) analyzing the immobilized polypeptide to detect the presence or absence of radioactivity from said radiolabeled antibody.

13. A Factor VIII antibody neutralizing product comprising polypeptides whose amino acid sequences include at least one fragment of human Factor VIII composed of amino acid residues 1649-2332 or a smaller fragment thereof, amino acid residues 1-740 or a smaller fragment thereof, amino acid residues 1690-2332 or a smaller fragment thereof, the amino acid residues 373-740 or a smaller fragment thereof, the amino acid residues or smaller fragments thereof in any combination.

14. A method of removing Factor VIII antibody inhibitors from blood of a patient exhibiting Factor VIII inhibitory activity, comprising

(a) immobilizing on a solid substrate a polypeptide according to claim 4;

(b) obtaining blood from said patient;

(c) contacting the immobilized polypeptide with the blood and removing the Factor VIII antibody inhibitors from said blood; and

(d) returning the antibody inhibitor free blood to the patient.

15. A method as defined in Claim 14, comprising

(a) separating plasma from the blood;

(b) contacting the immobilized polypeptide with the separated plasma and removing the Factor VIII antibody inhibitors from said plasma; and

(c) returning the antibody inhibitor free plasma and said blood to the patient.